# FASCICULE DE BREVET EUROPEEN

(11) **EP 0 770 407 B1**
(45) Date de publication et mention de la délivrance du brevet: **06.08.2003**
(21) Numéro de dépôt: 96402177.8
(22) Date de dépôt: 11.10.1996
(51) Int. Cl.: A61N 1/365, A61N 1/00

(54) **Dispositif médical implantable actif, notamment stimulateur cardiaque, à fonctionnement asservi et consommation réduite**
Aktive implantierbare medizinische Vorrichtung, insbesondere Herzschrittmacher, mit geregeltem Betrieb und reduziertem Energieverbrauch
Active implantable medical device, especially a cardiac pacemaker, with controlled operation and reduced power consumption

(30) Priorité: 13.10.1995 FR 9512051
(43) Date de publication de la demande: 02.05.1997
(73) Titulaire: ELA MEDICAL (Société anonyme), 92541 Montrouge (FR)
(72) Inventeur: Bonnet, Jean-Luc, 92170 Vanves (FR); Dubreuil, Anne, 92100 Boulogne (FR)
(74) Mandataire: Dupuis-Latour, Dominique

(56) Documents cités:
- EP-A- 0 191 404
- EP-A- 0 431 437
- EP-A- 0 541 338
- EP-A- 0 570 674
- EP-A- 0 616 819
- EP-A- 0 640 359
- WO-A-88/09684
- DE-A- 3 939 898
- US-A- 5 014 702
- US-A- 5 065 759

## Description

La présente invention concerne les "dispositifs médicaux implantables actifs" tels que définis par la directive 90/385/CEE du 20 juin 1990 du Conseil des communautés européennes, notamment les stimulateurs cardiaques ou défibrillateurs, dont le fonctionnement est asservi à un paramètre recueilli à l'aide d'un capteur.

À cet égard, bien que dans la suite de la description on fasse principalement référence au cas d'un stimulateur cardiaque, l'invention est applicable de façon générale à une très grande variété de dispositifs électroniques.

Ces appareils sont connus pour adapter leurs actions, par exemple la fréquence de stimulation dans le cas d'un stimulateur cardiaque, à une valeur mesurée ou calculée, telle qu'un paramètre représentatif des besoins métaboliques du porteur de l'appareil.

De multiples paramètres, tels que la ventilation-minute, la fréquence respiratoire, la saturation d'oxygène dans le sang, la température ou l'accélération ont été utilisés comme paramètres d'asservissement, en particulier dans le cas des stimulateurs cardiaques pour faire varier la fréquence instantanée de la stimulation cardiaque.

Certains procédés prévoient même, comme cela est décrit dans les US-A-5 014 702 et US-A-5 065 759, de combiner les informations issues de deux capteurs de types différents afin de profiter des avantages de chacun d'eux.

L'utilisation d'un ou de plusieurs capteurs d'asservissement concourt à l'amélioration du fonctionnement du stimulateur, en permettant à ce dernier de fonctionner d'une manière beaucoup plus proche des besoins métaboliques et physiologiques réels du patient porteur du dispositif implanté.

Toutefois, l'utilisation d'un ou, à plus forte raison, de plusieurs capteurs implique une consommation d'énergie supplémentaire tant en ce qui concerne les circuits matériels directement associés au transducteur (alimentation en tension, injection de courant, production et analyse du signal, etc.) que le logiciel exploitant les signaux produits, les microprocesseurs ou circuits spécifiques exécutant le logiciel de traitement étant typiquement des composants gros consommateurs d'énergie lorsqu'ils exécutent les algorithmes.

L'un des buts de l'invention est de réduire la consommation énergétique globale du dispositif en cherchant à ne mettre en service les circuits matériels et les logiciels directement associés aux capteurs (éléments que l'on désignera par la suite collectivement sous le vocable de "chaîne de mesure et de traitement" ou simplement "chaîne de mesure") que de façon intermittente afin d'économiser l'énergie de la pile et prolonger ainsi d'autant la durée de vie du dispositif implanté.

Des techniques ont déjà été proposées dans ce sens.

Ainsi, le DE-A-39 39 898 prévoit d'ajouter au dispositif un capteur supplémentaire spécifique (du type capteur d'activité), commandant la mise en ou hors service de la chaîne de mesure du capteur d'asservissement selon que ce capteur spécifique détecte ou non une activité du patient. Ce dispositif présente cependant l'inconvénient de devoir recourir à un capteur supplémentaire spécifique ayant pour seul objet la détection de l'activité ; outre l'accroissement de la complexité du dispositif, ce capteur, qui doit être en service en permanence, implique une consommation d'énergie supplémentaire qui grève le gain de consommation obtenu sur la chaîne de mesure du capteur d'asservissement.

Le WO-A-91/08020 prévoit de commander la mise en ou hors service de l'unique capteur par un circuit à boucle à verrouillage de phase ajusté sur le rythme circadien du patient. Si l'inconvénient de la proposition précédente est supprimé compte tenu de la faible consommation de la boucle, cette configuration présente l'inconvénient du caractère simplement prédictif de la commutation, qui ne prend pas en compte l'activité réelle du patient et n'ajuste qu'après coup les variations de son rythme circadien. En outre, ce dispositif ne prend en compte que le rythme jour/nuit, sans pouvoir intégrer des périodes d'inactivité plus courte, par exemple des repos diurnes.

L'un des buts de la présente invention est de remédier à ces difficultés, en proposant un dispositif médical implantable actif asservi, notamment un stimulateur cardiaque, susceptible de mettre en ou hors service la chaîne de mesure du capteur d'asservissement avec un minimum de moyens matériels et logiciels, donc en procurant une économie d'énergie importante, mais en étant pour autant capable de prendre en compte des variations d'activité même de brève durée et pouvant survenir à n'importe quel moment de la journée.

Le dispositif est du type générique connu, enseigné notamment par le DE-A-39 39 898 précité, et comportant : un premier capteur mesurant un paramètre servant au pilotage d'au moins une fonction du dispositif, notamment d'une fréquence de stimulation cardiaque ; une première chaîne de mesure et de traitement exploitant les signaux délivrés par ce premier capteur ; et des moyens pour mettre en ou hors service la première chaîne de mesure et de traitement en fonction de l'activité du patient porteur du dispositif.

Selon l'invention, la première chaîne de mesure et de traitement détermine un état d'activité du premier capteur, cet état étant susceptible de prendre, en fonction de critères prédéterminés, une valeur calculée représentative d'un état de repos du patient, et la première chaîne de mesure est adaptée à être mise hors service en fonction de l'état d'activité du premier capteur, lorsque cet état est établi à l'état de repos.

Très avantageusement, le dispositif comporte un second capteur mesurant un paramètre représentatif de l'activité du patient et une seconde chaîne de mesure et de traitement exploitant les signaux délivrés par ce second capteur, et la première chaîne de mesure est adaptée à être remise en service en fonction des signaux délivrés par le second capteur.

En variante, le dispositif peut comporter un compteur, incrémenté en fonction du temps écoulé après mise hors service de la première chaîne de mesure, et on remet en service la première chaîne de mesure en fonction de la valeur atteinte par le compteur.

Le premier capteur peut être soit un capteur d'effort, mesurant un paramètre à prépondérance physiologique et délivrant un signal fonction de l'effort développé par le patient, soit un capteur d'activité, en particulier un capteur mesurant un paramètre à prépondérance physique.

Dans une forme préférentielle de réalisation à deux capteurs comme précité, le premier capteur est un capteur d'effort, mesurant un paramètre à prépondérance physiologique et délivrant un signal fonction de l'état métabolique du patient, et le second capteur est un capteur d'activité, en particulier un capteur à faible temps de réponse mesurant un paramètre à prépondérance physique.

D'autres caractéristiques et avantages de l'invention apparaîtront à la lecture de la description détaillée ci-dessous, faite en référence à la figure unique annexée qui est un schéma par blocs fonctionnels d'un dispositif mettant en oeuvre les enseignements de l'invention.

On va décrire l'invention dans le cadre d'un stimulateur cardiaque asservi dans lequel un ou plusieurs capteurs servent au pilotage de la fréquence de stimulation cardiaque.

Cette application n'est cependant pas limitative, et l'invention peut s'appliquer, comme on le comprendra, à d'autres dispositifs médicaux implantables actifs dont une fonction est pilotée par un capteur.

De même, bien que l'on fera référence à un mode de réalisation préférentiel dans lequel le stimulateur est doublement asservi ― par un capteur d'effort et par un capteur d'accélération ― l'invention est également applicable à un dispositif à simple asservissement, l'idée-mère de l'invention reposant, comme on le comprendra, sur une "auto-déconnexion" du capteur d'asservissement, quelle que soit la manière dont ce capteur est remis en service ultérieurement : par un second capteur, comme on le décrira, par une temporisation ou un compteur, par le logiciel d'analyse des signaux cardiaques détectés ou toute autre commande intrinsèque.

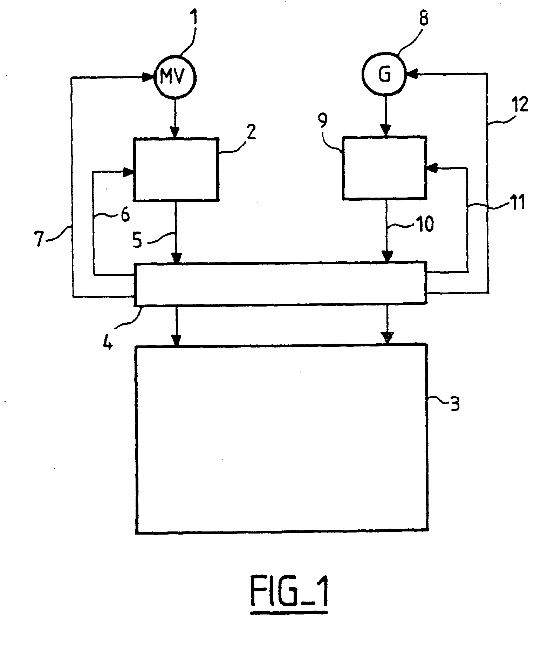

Sur le diagramme fonctionnel schématique de la figure 1, la référence 1 désigne le capteur d'asservissement principal du stimulateur. Ce capteur est associé à un ensemble de circuits 2, que l'on appellera "chaîne de mesure" et qui comprend l'ensemble des éléments physiques et logiques permettant de faire fonctionner le capteur et de délivrer un signal utilisable par le circuit principal 3 du stimulateur, comprenant notamment le microprocesseur de traitement des informations pour l'asservissement de la stimulation.

Dans cet exemple, le capteur est un capteur d'un paramètre physiologique qui est la "ventilation-minute" ("MV"). Mais l'invention est bien entendue applicable semblablement à d'autres paramètres physiologiques tels que ceux qui ont été indiqués dans l'introduction de la présente description ; tout autre recueil ou mesure d'un paramètre physiologique apte à être utilisé pour des fonctions telles qu'un asservissement de dispositif implantable actif (ou autre fonction) peut être substitué à la ventilation-minute sans sortir du cadre de la présente invention.

La mesure de la ventilation-minute est en elle-même bien connue ; elle est décrite notamment dans le document *Breath-by-Breath Minute Ventilation Measurement Can Provide a Fast Response,* par J. L. Bonnet, L. Krämer, M. Limousin, EUR J. C. P. E., 1994, Vol. 4, Abstract n° 329, et elle est mise en oeuvre dans les appareils *Chorus RM 7034* fabriqués par la société ELA Médical.

Par ailleurs, la mise en oeuvre du procédé décrit s'appuie de façon préférentielle sur une architecture composée d'un microprocesseur associé à des instructions de programmation en mémoire morte ROM et de circuits logiques et analogiques en eux-mêmes connus. Une telle structure est par exemple celle employée dans les stimulateurs cardiaques *Chorus* fabriqués par la société ELA Médical. Bien qu'elle ne présente pas les avantages de souplesse de mise en oeuvre de la solution à microprocesseur, une conception en circuits logiques dédiés et câblés est toutefois parfaitement envisageable et entre également dans le cadre de l'invention.

Pour la mise en oeuvre de l'invention, on peut prévoir un circuit d'interfaçage 4 (qui peut, en pratique, être intégré au circuit principal 3 si nécessaire) recevant en 5 les signaux de la chaîne de mesure du capteur et pouvant commander, respectivement en 6 et 7, la mise en service ou hors service de cette chaîne de mesure et si nécessaire de l'alimentation du capteur (par exemple l'injection de courant).

Avantageusement, il est prévu un second capteur 8, de préférence un capteur d'un paramètre non physiologique tel que l'accélération ("G") mesurée par un capteur tel que par exemple décrit dans le US-A-5 330 510. Ce capteur comporte également sa chaîne de mesure 9 et délivre en 10 un signal, transmis via le circuit d'interfaçage 4, au circuit principal 3 pour piloter l'asservissement de la stimulation. Un tel mode de réalisation à double capteur et double asservissement permet de profiter des avantages complémentaires de chacun des types de capteurs, le capteur MV donne une bonne représentation des besoins métaboliques du patient, mais présente un temps de réponse relativement long, tandis que le capteur G, dont le temps de réponse est court, permet de détecter rapidement un changement d'activité du patient et ainsi détecter notamment des débuts de phase d'effort. On notera cependant que la différence de temps de réponse entre les capteurs n'est pas forcément déterminante, la ventilation-minute étant par exemple connue comme étant un paramètre physiologique présentant en début d'effort une réponse rapide (c'est d'ailleurs la raison pour laquelle un simple asservissement peut, dans de nombreux cas, procurer des résultats satisfaisants).

Comme pour le capteur MV, le circuit d'interfaçage 4 peut commander, respectivement en 11 et 12, la mise en service ou hors service de la chaîne de mesure 9 et du capteur 8.

On va maintenant décrire diverses formes de mise en oeuvre de l'invention, pour la mise en ou hors service des capteurs MV et G.

Dans une première forme de réalisation, qui est la forme préférentielle, on utilise les deux capteurs MV et G, et le capteur principal est le capteur MV (c'est lui qui commande sa propre mise hors service, le capteur G en contrôlant la remise en service).

L'état d'activité du capteur MV, qui permet de déterminer si le patient est dans un état de repos ou un état d'activité, est avantageusement déterminé de la manière décrite dans la demande de brevet français FR-A-2 728 798, publié le 05.07.1996 et intitulée *Procédé de détermination d'un critère d'activité d'un capteur de mesure d'un paramètre d'asservissement dans un dispositif médical implantable actif* au nom de ELA Médical.

Le capteur MV effectue une mesure à chaque cycle respiratoire et calcule un moyenne à court terme VE₁₂₈ sur 128 cycles respiratoires, ainsi qu'une moyenne à long terme VE₂₄ₕ, déterminée sur 24 heures.

En ce qui concerne le capteur G, l'information d'accélération est mesurée typiquement toutes les 1,5625 seconde.

L'idée de base de l'invention consiste à mettre hors service le capteur MV lorsque celui-ci indique un état de repos établi pendant une certaine durée, par exemple pendant 4 x 128 cycles respiratoires, et à remettre ce capteur en service si le capteur G indique un état d'activité établi pendant un certain temps, par exemple pendant 32 x 1,5625 secondes.

L'algorithme général est le suivant :
si capteur MV en service
   tous les 128 cycles respiratoires
      si VE₁₂₈ < VE₂₄ₕ
         alors incrémenter (Compteur_Repos_MV)
            si Compteur_Repos_MV = 4
               alors mettre capteur MV hors service
            sinon rien
      sinon Compteur_Repos_MV = 0
sinon [c'est-à-dire si capteur MV hors service]
   tous les 4 cycles G
      si État_Capteur_G ≠ Repos
         alors incrémenter (Compteur_Activité_G)
            si Compteur_Activité_G = 8
               alors mettre capteur MV en service
            sinon rien
      sinon Compteur_ Activité_G = 0

En d'autres termes, le capteur MV (capteur 1 et sa chaîne de mesure 2) est mis hors service dès qu'une phase de repos ventilatoire est confirmée sur quelques minutes ; il est remis en service dès que le capteur G détecte une activité prolongée, avec un temps de remise en fonctionnement de l'ordre de 20 à 30 secondes (10 à 15 secondes pour obtenir des échantillons fiables, puis quatre cycles respiratoires pour obtenir la première moyenne nécessaire à l'asservissement).

Avantageusement (mais non nécessairement), on peut éviter de laisser le capteur G fonctionner en permanence lors des périodes d'inactivité du patient.

L'algorithme général est alors le suivant :
si État_Capteur_G = Repos
   alors
      si capteur MV en service
         alors mettre capteur G en service 1 échantillon sur 3
      sinon
         alors mettre capteur G en service 1 échantillon sur 10
sinon
   alors mettre capteur G en service en permanence

En d'autres termes, lorsque le capteur MV est en service, si aucune activité n'est détectée par le capteur G on éteint celui-ci deux cycles sur trois et, dès qu'une activité est suspectée, on repasse en fonctionnement permanent (le retard maximal induit par ce principe pour détecter le début d'effort étant de 4,7 secondes). En revanche, lorsque le capteur MV est hors service (par exemple pendant une phase de sommeil), si aucune activité n'est détectée par le capteur G, on met celui-ci hors service neuf cycles sur dix et, ici encore, dès qu'une activité est suspectée on repasse en fonctionnement permanent (le retard maximal induit étant alors de 15,6 secondes).

Dans une autre forme de mise en oeuvre de l'invention, cependant moins avantageuse, les rôles des capteurs MV et G sont inversés, le capteur G étant le capteur principal (celui susceptible de se mettre de lui-même hors service), et le capteur MV contrôlant la remise en service du capteur G. Les différents algorithmes ci-dessus peuvent être appliqués à cette autre forme de réalisation, *mutatis mutandis.*

On notera cependant que, du point de vue physiologique, cette dernière solution est moins satisfaisante car le temps de mise en fonctionnement du capteur MV pénalise les performances globales du dispositif. En outre, le capteur MV étant celui qui présente la consommation la plus élevée (du fait notamment de son fonctionnement par injection de courant et du logiciel plus complexe de la chaîne de mesure), il est généralement préférable de le mettre hors service en premier.

## Revendications

1. Un dispositif médical implantable actif susceptible d'un fonctionnement asservi, notamment un stimulateur cardiaque, ce dispositif comportant :
- un premier capteur (1) mesurant un paramètre servant au pilotage d'au moins une fonction du dispositif, notamment d'une fréquence de stimulation cardiaque,
- une première chaîne de mesure et de traitement (2) exploitant les signaux délivrés par ce premier capteur, et
- des moyens (4) pour mettre en ou hors service la première chaîne de mesure et de traitement en fonction de l'activité du patient porteur du dispositif,
**caractérisé par le fait que**
- la première chaîne de mesure et de traitement détermine un état d'activité du premier capteur, cet état étant susceptible de prendre, en fonction de critères prédéterminés, une valeur calculée représentative d'un état de repos du patient, et
- la première chaîne de mesure est adaptée à être mise hors service en fonction de l'état d'activité du premier capteur, lorsque cet état est établi à l'état de repos.

2. Le dispositif de la revendication 1, comportant en outre :
- un second capteur (8) mesurant un paramètre représentatif de l'activité du patient,
- une seconde chaîne de mesure (9) et de traitement exploitant les signaux délivrés par ce second capteur,
et dans lequel :
- la première chaîne de mesure est adaptée à être remise en service en fonction des signaux délivrés par le second capteur.

3. Le dispositif de la revendication 1, comportant en outre :
- un compteur, incrémenté en fonction du temps écoulé après mise hors service de la première chaîne de mesure,
et dans lequel :
- on remet en service la première chaîne de mesure en fonction de la valeur atteinte par le compteur.

4. Le dispositif de la revendication 1, dans lequel le premier capteur (1) est un capteur d'effort, mesurant un paramètre à prépondérance physiologique et délivrant un signal fonction de l'effort développé par le patient.

5. Le dispositif de la revendication 1, dans lequel le premier capteur est un capteur d'activité, en particulier un capteur mesurant un paramètre à prépondérance physique.

6. Le dispositif des revendications 2 et 4 prises en combinaison, dans lequel le premier capteur (1) est un capteur d'effort, mesurant un paramètre à prépondérance physiologique et délivrant un signal fonction de de l'état métabolique du patient, et le second capteur (8) est un capteur d'activité, en particulier un capteur à faible temps de réponse mesurant un paramètre à prépondérance physique.

7. Le dispositif des revendications 2 et 3 prises en combinaison, dans lequel, si le second capteur est à l'état de repos, on fait fonctionner ce capteur de façon intermittente à une fréquence de mise en service d'autant plus faible que le premier capteur est mis hors service.

## Patentansprüche

1. Aktive, implantierbare medizinische Vorrichtung, geeignet zum gesteuerten Funktionieren, insbesondere ein Herzschrittmacher, wobei die Vorrichtung aufweist:
- einen ersten Sensor (1), der einen Parameter misst, welcher der Steuerung zumindest einer Funktion der Vorrichtung dient, insbesondere einer Herzstimulationsfrequenz,
- einen ersten Mess- und Verarbeitungsabschnitt (2), der die durch diesen ersten Sensor gelieferten Signale auswertet, und
- Mittel (4) zum Ein- oder Ausschalten des ersten Mess- und Verarbeitungsabschnitts in Abhängigkeit der Aktivität des Patienten, der die Vorrichtung trägt,
**dadurch gekennzeichnet, dass**
- der erste Mess- und Verarbeitungsabschnitt einen Aktivitätszustand des ersten Sensors bestimmt, wobei dieser Zustand geeignet ist, in Abhängigkeit von vorbestimmten Kriterien, einen berechneten Wert anzunehmen, der repräsentativ ist für einen Ruhezustand des Patienten, und
- der erste Messabschnitt angepasst ist, außer Betrieb gesetzt zu werden in Abhängigkeit des Aktivitätszustands des ersten Sensors, wenn dieser Zustand in den Ruhezustand gesetzt ist.

2. Vorrichtung gemäß Anspruch 1, weiter aufweisend:
- einen zweiten Sensor (8), der einen Parameter misst, welcher repräsentativ für die Aktivität des Patienten ist,
- einen zweiten Mess- (9) und Verarbeitungsabschnitt, der die durch diesen zweiten Sensor gelieferten Signale auswertet,
und bei welcher:
- der erste Messabschnitt angepasst ist, wieder in Betrieb gesetzt zu werden in Abhängigkeit der durch den zweiten Sensor gelieferten Signale.

3. Vorrichtung gemäß Anspruch 1, weiter aufweisend:
- einen Zähler, der in Abhängigkeit der Zeit inkrementiert wird, welche verstrichen ist nach Außerbetriebnahme des ersten Messabschnitts,
und bei welcher:
- der erste Messabschnitt wieder in Betrieb genommen wird in Abhängigkeit des durch den Zähler erreichten Werts.

4. Vorrichtung gemäß Anspruch 1, bei welcher der erste Sensor (1) ein Belastungssensor ist, der einen Parameter zur physiologischen Vorherrschaft misst und ein Funktionssignal der durch den Patienten entwickelten Anstrengung abgibt.

5. Vorrichtung gemäß Anspruch 1, bei welcher der erste Sensor ein Aktivitätssensor ist, insbesondere ein Sensor, der einen Parameter zur physischen Vorherrschaft misst.

6. Vorrichtung gemäß Ansprüchen 2 und 4, in Kombination genommen, bei welcher der erste Sensor (1) ein Belastungssensor ist, der einen Parameter zur physiologischen Vorherrschaft misst und ein Funktionssignal des metabolischen Zustands des Patienten abgibt, und der zweite Sensor (8) ein Aktivitätssensor ist, insbesondere ein Sensor mit schwacher Antwortzeit, der einen Parameter zur physischen Vorherrschaft misst.

7. Vorrichtung gemäß Ansprüchen 2 und 3, in Kombination genommen, bei welcher, wenn der zweite Sensor im Ruhezustand ist, man diesen Sensor funktionieren lässt in intermittierender Weise mit einer Inbetriebnahme-Frequenz, die umso schwächer ist, je mehr der erste Sensor außer Betrieb genommen wird.

## Claims

1. An active implantable medical device having a function which may be enslaved to, in particular a cardiac pacemaker, said device including:
- a first sensor (1) measuring a parameter used for driving at least one function of the device, in particular a cardiac pacing frequency,
- a first measurement and processing circuit (2) which is responsive to the signals issued by said first sensor, and
- means (4) for switching in or out the first measurement and processing circuit as a function of the activity of the patient bearing the device,
**characterized in that**:
- the first measurement and processing circuit determines a state of activity of the first sensor, wherein said state may be set, as a function of predetermined criteria, to a calculated value representative of a state of rest of the patient, and
- the first measurement circuit is adapted to be switched out as a function of the state of activity of the first sensor, when said state is set to the state of rest.

2. The device of claim 1, further including:
- a second sensor (8) measuring a parameter representative of the activity of the patient,
- a second measurement and processing circuit (9) which is responsive to the signals issued by said second sensor,
and wherein:
- the first measurement circuit is adapted to be switched in back as a function of the signals issued by the second sensor.

3. The device of claim 1, further including:
- a counter, which is incremented as a function of the time elapsed after the first measurement circuit is switched in,
and wherein:
- the first measurement circuit is switched in back as a function of the value reached by the counter.

4. The device of claim 1, wherein the first sensor (1) is an effort sensor measuring a mainly physiological parameter and issuing a signal as a function of the effort developed by the patient.

5. The device of claim 1, wherein the first sensor is an activity sensor, in particular a sensor measuring a mainly physical parameter.

6. The device of claims 2 and 4 taken in combination, wherein the first sensor (1) is an effort sensor measuring a mainly physiological parameter and issuing a signal which is a function of the metabolic state of the patient, and the second sensor (8) is an activity sensor, in particular a low response-time sensor measuring a mainly physiological parameter.

7. The device of claims 2 and 3 taken in combination, wherein, if the first sensor (1) is in the rest state, said sensor is operated intermittently at a working frequency which is all the lower as the first sensor is switched out.
